# EUROPEAN PATENT SPECIFICATION

(11) **EP 4 125 826 B1**
(45) Date of publication and mention of the grant of the patent: **11.06.2025**
(21) Application number: 21701054.5
(22) Date of filing: 12.01.2021
(51) Int. Cl.: A61K 9/51, A61K 31/405, A61P 29/00

(54) **NANO-DRY MELTING**
NANO-TROCKENSCHMELZE
FUSION NANOMÉTRIQUE SÈCHE

(30) Priority: 23.03.2020 EP 20164850
(43) Date of publication of application: 08.02.2023
(73) Proprietor: Bayer Aktiengesellschaft, 51373 Leverkusen (DE)
(72) Inventor: HEUMANN, Roman, 40764 Langenfeld (DE); HOHEISEL, Werner, 51061 Köln (DE); NÜBOLDT, Christoph, 50969 Köln (DE)
(74) Representative: BIP Patents
(86) International application number: PCT/EP2021/050440
(87) International publication number: WO 2021/084139

(56) References cited:
- EP-A1- 2 601 935
- WO-A1-2016/016665
- WO-A1-2018/229626
- US-A1- 2011 306 539
- US-A1- 2019 021 997
- SHRAWAN BAGHEL ET AL: "Polymeric Amorphous Solid Dispersions: A Review of Amorphization, Crystallization, Stabilization, Solid-State Characterization, and Aqueous Solubilization of Biopharmaceutical Classification System Class II Drugs", JOURNAL OF PHARMACEUTICAL SCIENCES, vol. 105, no. 9, 23 January 2016 (2016-01-23), US, pages 2527 - 2544, XP055430383, ISSN: 0022-3549, DOI: 10.1016/j.xphs.2015.10.008

## Description

Amorphous solid dispersions (ASDs) are being used with increasing frequency for poorly soluble pharmaceutical compounds. In an ASD, the active ingredient (AI) is molecularly dispersed in a polymer matrix and thus stabilized in an amorphous state. This system is only thermodynamically stable, if the polymer is capable of completely dissolving the active ingredient.

In light of this it is the object of the present invention to provide an improved method for the preparation of an amorphous solid dispersion comprising the steps of
- providing an aqueous suspension at a temperature between 253 K und 323 K comprising nanoparticles having a solubility in the aqueous suspension of less than 10g/l, water and at least one polymer,
- nano-dry-melting of the aqueous suspension comprising nano-particles for a time span between 0.1 seconds and 300 seconds, at a temperature between
   a) 20K below the glass transition temperature - as determined with DSC according to DIN EN ISO 11357-2 at a heating rate of 10 K/min - of the solid components of the aqueous suspension comprising nano-particles or 20K below the glass transition temperature of the at least one polymer, depending on what is higher,
   b) the decomposition temperature of the at least one polymer or the decomposition temperature of the nano-particles, depending which is lower
   to form the amorphous solid dispersion.

It should be noted that a person skilled in the art is well aware of the fact that the time needed to form an ASD via nano-dry-melting is dependent on various factors e.g. the melting temperature of the active ingredient, T_{g} of polymer(s) and active ingredient, additives and solubility of the active ingredient in the polymer(s). In general, the higher the temperature the shorter the required time span and the smaller the particles the shorter the required time span. Below a characteristic temperature, which is usually the lowest T_{g} of the applied materials, an ASD will not be formed at all within a reasonable time span.

The melting temperature can be determined according to DIN EN ISO 11357-3 at a heating rate of 10 K/min.

Using the method as described herein, neither active ingredient nor matrix forming polymers and optionally surfactants have to be dissolved in an organic solvent or solvent mixture. Thus, it is no longer necessary to use one of only a few by the European Medical Agency permitted ICH-3 classified solvents, which additionally must have a high vapour pressure in order to be able to remove them sufficiently quick and at temperatures that are not too high during the drying process. Thereby also the problem is circumvented that some modern active ingredients are only slightly soluble in the typically used solvents ethanol and/or acetone and, if necessary, alternatives such as butanone, so that large quantities of these solvents are required. During the evaporation process, these solvents should or must be recovered and reprocessed for environmental reasons, which causes further effort and costs. In addition, a further disadvantage of these solvent-based methods, i.e. the fact that it has to be ensured that no residual solvent remains in the later product or must be below the usually low ICH limits, is overcome.

Likewise, the method described herein does not require relatively long residence time (which can be many minutes in order to ensure full dissolution of active ingredient in polymer) at elevated temperature as it is the case for example in an extruder. This elevated temperature results in thermal stress for the active ingredient and/or excipient(s). In addition, restrictions such as that the melting temperature of the active ingredient and the glass transition temperature of the polymer (or melting temperature for crystalline or semi-crystalline polymers) must not be too far apart, so that the dissolution process can take place in a technically reasonable period of time do not apply. Moreover, other technical necessities of an extrusion process can be omitted e.g. that after extrusion the resulting extrusion strand has to be crushed with a further grinding process to obtain a flowable powder on the one hand and a powder that does not take too long to dissolve in aqueous media on the other. The latter is necessary because the extruded material is very compact and, despite its increased solubility in water in principle, would therefore take too long time to get completely dissolved in the gastrointestinal tract.

A person skilled in the art is aware of the different methods for generating amorphous solid dispersion known in the art:
Solvent-based processes: The active ingredient and usually at least one polymer are molecularly dissolved in a common organic solvent and then the amorphous solid dispersion is obtained by a drying process. This process can be carried out in various ways, and is for example based on spray drying, contact drying, freeze drying or methods based on fluidized beds in which the solvent is removed from the product. In industrial production, spray drying also uses variations of this basic process, in which the dissolved mixture of active ingredient, polymer, organic solvent and optionally further components is sprayed, e.g. on carrier particles (usually in the range of several 10 µm to over 1 mm in size) consisting of one type of sugar, cellulose, crosscarmellose or other inactive materials (spray granulation). This can also be done e.g. in a fluidized bed spray granulation plant or other suitable equipment. This results in directly flowable powders of defined composition from the amorphous solid dispersion and, if necessary, the carrier material. In new, mostly still experimental processes, sometimes inorganic carrier materials such as mesoporous silica are also used, in which the active ingredient solution is introduced into fine pores of the carrier material, e.g. by capillary forces, which is then dried. There are also other solvent-based processes. Disadvantageous of these methods are the use of organic solvents which are necessary in sometimes large relative amounts, depending on the solubility of the solutes involved. The use of organic solvents bears inherent risks regarding residual solvents in the product but also regarding environmental, safety, economic or handling reasons.

Alternatively heat-based processes can be used. In these processes, the active ingredient, which may have been micronized, i.e. the average particle diameters were reduced to the micrometer range (usually 1 - 50 µm), beforehand, is dry-mixed as a crystalline solid with polymer powder and then subjected to a heat and mixing process. An extruder is usually used for this e.g. a twin-screw extruder. In this process, the mixture is heated to temperatures close to or above the glass transition temperature of the amorphous or semi-crystalline polymer, so that a polymer compound is formed with a viscosity that is sufficiently low to be treated by the applied extruder. Further, the temperature must be close or above the melting point of the AI so that the active ingredient is then dissolved into the polymer matrix. The kneading and mixing of the materials used by the extruder screws supports this process. With crystalline or semi-crystalline polymers, the extruder temperature must be adapted to the temperature and shear stress dependent viscosity of this polymer. Disadvantageous of heat-based processes is the necessity of high temperatures that must be applied over a long period of time of usually more than 10 min to ensure a sufficient amorphization and mixing of the ingredients ("The application of temperature-composition phase diagrams for hot melt extrusion processing of amorphous solid dispersions to prevent residual crystallinity" by D. E. Moseson et al., Int. J. Pharm. 2018, 553, 454-466). This method incorporates the risk of degradation or alteration of the AI and/or excipient(s) due to long exposure time to elevated temperature and high shear forces. This generally can impact the product quality ("How changes in molecular weight and PDI of a polymer in amorphous solid dispersions impact dissolution performance" by C. Auch et al., Int. J. Pharm. 2019, 556, 372-382.

As used herein, the term "amorphous" refers to compositions which are substantially non-crystalline. The amorphous nature of a composition can be determined e.g. by X-ray powder diffraction (XRPD). As sharp peaks in the XRPD pattern indicate the presence of crystalline material, a composition is "amorphous" when there are no sharp peaks observed in its XRPD pattern, only broad halos. In other words when scanned with a X-ray diffractometer the amorphous compositions do not show a discernable X-ray diffraction pattern when scanned from, for example, 0.5 to 55 degrees.

The term "solid dispersion" is employed synonymously with the terms "amorphous solid dispersion" and "solid solutions" and refers herein to a situation where a first component is completely dispersed at molecular level in at least one second component. Said first component is not dissolved in said at least one second component but is distributed - preferably evenly distributed - throughout the at least one second component. In this respect it should be noted that if an active ingredient such as a pharmaceutical agent is said first component the dispersion is termed "amorphous solid dispersion" if the active ingredient is amorphous.

It should be noted that in general the aqueous suspension comprising nano-particles only comprises water as solvent. As specified below a person skilled in the art knows situations where it is appropriate to add components such as surfactants etc., however, an aqueous suspension comprising nano-particles differs from a suspension comprising nano-particles in that it does not comprise organic solvents.

As used herein, the term "particle" refers to a solid, gel, or semisolid material having a relatively small size characterized in that as nano-particles - e.g. as active ingredient nanoparticles - said particles have a solubility of less than 10g/l in a suspension comprising water, matrix forming polymer(s) and optionally surfactants as determined e.g. by HPLC.

As used herein the term "nano" refers to particles having an average particle size - i.e. d50 of the distribution - of ≥ 10 nm to ≤ 999 nm.

Herein the nanoparticles have a particle size distribution of d90 < 2µm preferably < 1µm especially preferred < 500 nm and d50 < 1 µm preferably < 500 nm especially preferred < 200 nm. The particle size distribution can be determined by methods known in the art such as laser diffraction or Dynamic Light Scattering (DLS). The term d50 or d90 means that 50% or 90% of the total product volume consists of particles smaller than the given value for d50 or d90. The given particle size corresponds to the hydrodynamic particle size when determined by DLS and for both methods an equivalent size for spheres is given and meant here.

The term "glass transition temperature" as used herein refers to the temperature T_{g} of a material and characterizes the range of temperatures over which this glass transition occurs. It is determined herein with differential scanning calorimetry (DSC) according to DIN EN ISO 11357-2 at a heating rate of 10 K/min. The glass-liquid transition, or glass transition, is the gradual and reversible transition in amorphous materials (or in amorphous regions within semi-crystalline materials) from a hard and relatively brittle "glassy" state into a viscous or rubbery state as the temperature is increased. An amorphous solid that exhibits a glass transition is called a glass. The reverse transition, achieved by supercooling a viscous liquid into the glass state, is called vitrification.

As used herein the term "solubility in the liquid part of the aqueous suspension" refers to the solubility of the nanoparticles e.g. the active ingredient nanoparticles in a solution comprising water, matrix forming polymer(s) and optionally surfactants as determined e.g. by HPLC. A person skilled in the art is aware of the fact that in order to determine the solubility the nanoparticles e.g. the active ingredient nanoparticles, are added to the aqueous suspension comprising water, matrix forming polymer(s) and optionally surfactants.

In the method described herein the aqueous suspension is provided at a temperature between 253K and 323K thus the solubility of the nano-particles of less than 10g/l in the liquid phase of the aqueous suspension is given at this temperature range.

As used herein the term "Nano-Melt-Drying" refers to the fact that the nano-particles are dissolved in the polymer during the drying step due to their nano-properties, i.e. their small size and is supported by applying heat. This dissolution is extremely fast i.e. between 0.1 seconds (sec) and 300 seconds. The smaller the particle size and the higher the applied temperature the faster is the process of dissolving the nanoparticles in the polymer. The term "Nano-Melt-Drying" can be used synonymously with "Nano-Dry-Melting", "Dry-Melting" or "Melt-Drying" even though the term "Nano-Dry-Melting" best describes the process.

A skilled person is aware that in case that more than one polymer is used, the T_{g} refers to the T_{g} of the polymer mixture or the lowest T_{g} of one of the polymers.

Suitable methods for Nano-Dry-Melting can be selected from the group consisting of spray drying, spray granulation, fluidized bed drying systems, contact drying, extrusion processes and drying by electromagnetic radiation.

In general, a person skilled in the art is aware of the different methods for drying compositions comprising nanoparticles such as spray drying or spray granulation. In this process, the suspension comprising nanoparticles e.g. the aqueous suspension comprising nano-particles is introduced into a spray dryer and subjected to the drying process normally used in this apparatus. Optionally, also micrometer sized carrier particles like lactose or others can additionally be used during drying process as it is usually done in solvent based drying processes and well described in the state of the art.

However, in the process described herein the inlet temperature of the drying gas is adjusted so that it is at or above the glass transition temperature of the polymer used. The temperature of the drying gas should be as high as possible, but well below the decomposition temperature of the active substance or additional components. The respective decomposition temperatures of AI and polymer can be determined beforehand by dynamic differential calorimetry (DSC) or thermogravimetrical analysis (TGA). Due to the applied temperature, the active ingredient is dissolved into the polymer during the drying phase, resulting in an amorphous solid dispersion (ASD). This ASD is formed during the time the material remains in the spray drying system.

The necessary temperature is between
a) 20K below the glass transition temperature as determined with DSC according to DIN EN ISO 11357-2 at a heating rate of 10 K/min of the solid components of the aqueous suspension comprising nano-particles or a suspension comprising nano-particles or 20K below the glass transition temperature of the at least one polymer, depending on what is higher,
b) the decomposition temperature of the at least one polymer or the decomposition temperature of the nano-particles, depending which is lower.

In general, when using a spray dryer or a fluidized bed dryer the outlet temperature is between
a) 20K below the glass transition temperature as determined with DSC according to DIN EN ISO 11357-2 at a heating rate of 10 K/min of the solid components of the aqueous suspension comprising nano-particles or a suspension comprising nano-particles or 20K below the glass transition temperature of the at least one polymer, depending on what is higher,
b) the decomposition temperature of the at least one polymer or the decomposition temperature of the nano-particles, depending which is lower.

A person skilled in the art is aware of the fact that the outlet temperature is used herein in order to draw conclusions about the maximum product temperature. In case of an adiabatic process, which can account for differing forms of spray drying or a fluidized bed drying apparatuses the outlet temperature corresponds to the maximum product temperature. In case of non-adiabatic processes the maximum product temperature can be determined via measuring the actual outlet temperature and using e.g. Mollier diagrams. A person skilled in the art knows from the set up of a given process or apparatus whether said process or apparatus is run under adiabatic conditions and can hence always determine the maximum product temperature of a given situation.

In a preferred embodiment when using a spray dryer or a fluidized bed dryer the outlet temperature is between
a) the glass transition temperature as determined with DSC according to DIN EN ISO 11357-2 at a heating rate of 10 K/min of the solid components of the aqueous suspension comprising nanoparticles or a suspension comprising nano-particles or the glass transition temperature of the at least one polymer, depending on what is higher,
b) the decomposition temperature of the at least one polymer or the decomposition temperature of the nano-particles, depending which is lower.

In an even more preferred embodiment when using a spray dryer or a fluidized bed dryer the outlet temperature is between
a) 20K above the glass transition temperature as determined with DSC according to DIN EN ISO 11357-2 at a heating rate of 10 K/min of the solid components of the aqueous suspension comprising nano-particles or a suspension comprising nano-particles or 20K above the glass transition temperature of the at least one polymer, depending on what is higher,
b) the decomposition temperature of the at least one polymer or the decomposition temperature of the nano-particles, depending which is lower.

The advantage of this method is that the melting temperature of a crystalline active ingredient does not always have to be reached for the dissolution process described.

At the outlet of the spray dryer the dry amorphous solid dispersion powder in the desired composition is discharged from the plant. A plant similar to the spray dryer can also be used as an installation, e.g. spray granulation or fluidized bed spray granulation. The particles have to be present as nanoparticles in suspension as input material to increase the dissolution rate of the nanoparticles into the excipient polymer in such a way that the amorphization and dissolution process is fast enough to take place in the short residence time of the compound in the plant which - as described again below - occurs only for a time span between 0.1 seconds and 300 seconds, preferably for a time span between 0.1 seconds and 180 seconds, more preferably for a time span between 0.1 seconds and 120 seconds and most preferred for a time span between 0.1 seconds and 60 seconds.

In addition, contact drying can be used for drying an aqueous suspension comprising nano-particles or a suspension comprising nano-particles such roller drying or similar processes in which the suspension is brought into contact with a heated surface. In this process, the aqueous suspension comprising nanoparticles or the suspension comprising nano-particles is placed on a hot surface so that the aqueous liquid evaporates and the nanoparticles dissolve into the polymer in the hot environment. The drying process can be additionally supported by an applied vacuum. The pressure should not be below the vapour pressure of water at the given temperature in order to prevent so-called flash evaporation of the suspension when it is introduced into the vacuum chamber. The temperature of the contact surface should be in the range of the glass transition temperature of the polymer or polymer mixture used or above, but significantly below the decomposition temperature of the active ingredient or the auxiliary materials. In general, the applied temperature should be in a range as described for the outlet temperature of the spray or fluidized bed drying or granulation. An example of a plant in which such a drying process can take place is a vacuum roller dryer. Here the suspension is dried on a slowly rotating roller brought to the required temperature and is scraped off this roller with a knife as a dried ASD layer and collected in a container. A further example is an extruder which has a series of degassing ports to remove the water vapour that is generated during the drying process. An advantage of this process compared to a usual hot melt extrusion process is that the extruder is only used to remove the water and to dissolve the nano-particles e.g. the active ingredient nanoparticles in the polymer(s) due to the applied heat, resulting in a dry powder leaving the extruder. The temperature in at least one zone within the extruder is adjusted in correspondence to the temperature at the outlet of the spray drying process as described above i.e. the temperature is between
a) 20K below the glass transition temperature as determined with DSC according to DIN EN ISO 11357-2 at a heating rate of 10 K/min of the solid components of the aqueous suspension comprising nano-particles or the suspension comprising nano-particles or 20K below the glass transition temperature as determined with DSC according to DIN EN ISO 11357-2 at a heating rate of 10 K/min of the at least one polymer, depending on what is higher,
b) the decomposition temperature of the at least one polymer or the decomposition temperature of the nano-particles, depending which is lower.

In a preferred embodiment the temperature in at least one zone within the extruder is between
a) the glass transition temperature as determined with DSC according to DIN EN ISO 11357-2 at a heating rate of 10 K/min of the solid components of the aqueous suspension comprising nanoparticles or a suspension comprising nano-particles or the glass transition temperature of the at least one polymer, depending on what is higher,
b) the decomposition temperature of the at least one polymer or the decomposition temperature of the nano-particles, depending which is lower.

In an even more preferred embodiment the temperature in at least one zone within the extruder is between
a) 20K above the glass transition temperature as determined with DSC according to DIN EN ISO 11357-2 at a heating rate of 10 K/min of the solid components of the aqueous suspension comprising nano-particles or a suspension comprising nano-particles or 20K above the glass transition temperature of the at least one polymer, depending on what is higher,
b) the decomposition temperature of the at least one polymer or the decomposition temperature of the nano-particles, depending which is lower.

Further, electromagnetic radiation can be used for nano-dry-melting. One not limiting example for this process is the use of microwaves, like it is used e.g. in a microwave oven. The temperature range in this process should be adjusted in correspondence to the temperature at the outlet of the spray or fluidized bed dryer as described above i.e. the temperature is between
a) 20K below the glass transition temperature as determined with DSC according to DIN EN ISO 11357-2 at a heating rate of 10 K/min of the solid components of the aqueous suspension comprising nano-particles or a suspension comprising nano-particles or 20K below the glass transition temperature of the at least one polymer, depending on what is higher,
b) the decomposition temperature of the at least one polymer or the decomposition temperature of the nano-particles, depending which is lower.

In a preferred embodiment when using electromagnetic radiation, the temperature is between
a) the glass transition temperature as determined with DSC according to DIN EN ISO 11357-2 at a heating rate of 10 K/min of the solid components of the aqueous suspension comprising nanoparticles or a suspension comprising nano-particles or the glass transition temperature of the at least one polymer, depending on what is higher,
b) the decomposition temperature of the at least one polymer or the decomposition temperature of the nano-particles, depending which is lower.

In an even more preferred embodiment, when using electromagnetic radiation, the temperature between
a) 20K above the glass transition temperature as determined with DSC according to DIN EN ISO 11357-2 at a heating rate of 10 K/min of the solid components of the aqueous suspension comprising nano-particles or a suspension comprising nano-particles or 20K above the glass transition temperature of the at least one polymer, depending on what is higher,
b) the decomposition temperature of the at least one polymer or the decomposition temperature of the nano-particles, depending which is lower.

Another example comprises the use of electromagnetic radiation of higher frequency, like it is emitted e.g. by lasers, by discharge lamps or other light sources in the UV, visible or IR frequency range, which can be used for Nano-Dry-Melting. Again, the power of the electromagnetic radiation should be adjusted so that the temperature exposure of the nanoparticle containing compound is in correspondence with the outlet of the spray or fluidized bed dryer as described above i.e. the temperature is between
a) 20K below the glass transition temperature as determined with DSC according to DIN EN ISO 11357-2 at a heating rate of 10 K/min of the solid components of the aqueous suspension comprising nano-particles or the suspension comprising nano-particles or 20K below the glass transition temperature of the at least one polymer, depending on what is higher,
b) the decomposition temperature of the at least one polymer or the decomposition temperature of the nano-particles, depending which is lower.

In a preferred embodiment when using electromagnetic radiation of higher frequency, the temperature is between
a) the glass transition temperature as determined with DSC according to DIN EN ISO 11357-2 at a heating rate of 10 K/min of the solid components of the aqueous suspension comprising nanoparticles or a suspension comprising nano-particles or the glass transition temperature of the at least one polymer, depending on what is higher,
b) the decomposition temperature of the at least one polymer or the decomposition temperature of the nano-particles, depending which is lower.

In an even more preferred embodiment, when using electromagnetic radiation of higher frequency, the temperature is between
a) 20K above the glass transition temperature as determined with DSC according to DIN EN ISO 11357-2 at a heating rate of 10 K/min of the solid components of the aqueous suspension comprising nano-particles or a suspension comprising nano-particles or 20K above the glass transition temperature of the at least one polymer, depending on what is higher,
b) the decomposition temperature of the at least one polymer or the decomposition temperature of the nano-particles, depending which is lower.

A not limiting example for which such a process could be used is the powder bed fusion process e.g. applied in 3D printing of drug containing solid dosage forms.

In one embodiment of the method the processes of drying and melting can be separated in two consecutively carried out independent process steps. Thus, as a non-limiting example, in a first step an aqueous suspension comprising nanoparticles or a suspension comprising nano-particles can be dried with a method described above, which means e.g. by spray drying, spray granulation, fluidized bed spray granulation, contact drying, electromagnetic drying or similar with process parameters that preserve the nanoparticles during drying resulting in a dry compound comprising nanoparticles. Freeze drying is another option for drying here. This can be achieved by applying temperatures below the glass transition temperature as determined with DSC according to DIN EN ISO 11357-2 at a heating rate of 10 K/min of the solid component of the aqueous suspension comprising nanoparticles or a suspension comprising nano-particles or the glass transition temperature of the polymer(s), depending on what is higher. In a second step the obtained dry compound comprising nanoparticles will be subjected to the same or to a different process step as described above but with a temperature profile that is described for nano-dry-melting by means of the spray-drying process i.e. the temperature is between
a) 20K below the glass transition temperature as determined with DSC according to DIN EN ISO 11357-2 at a heating rate of 10 K/min of the solid components of the aqueous suspension comprising nano-particles or a suspension comprising nano-particles or 20K below the glass transition temperature of the at least one polymer, depending on what is higher,
b) the decomposition temperature of the at least one polymer or the decomposition temperature of the nano-particles, depending which is lower.

In a preferred embodiment when using freeze drying the temperature is between
a) the glass transition temperature as determined with DSC according to DIN EN ISO 11357-2 at a heating rate of 10 K/min of the solid components of the aqueous suspension comprising nanoparticles or a suspension comprising nano-particles or the glass transition temperature of the at least one polymer, depending on what is higher,
b) the decomposition temperature of the at least one polymer or the decomposition temperature of the nano-particles, depending which is lower.

In an even more preferred embodiment, when using freeze drying the temperature is between
a) 20K above the glass transition temperature as determined with DSC according to DIN EN ISO 11357-2 at a heating rate of 10 K/min of the solid components of the aqueous suspension comprising nano-particles or a suspension comprising nano-particles or 20K above the glass transition temperature of the at least one polymer, depending on what is higher,
b) the decomposition temperature of the at least one polymer or the decomposition temperature of the nano-particles, depending which is lower.

As a non-limiting example for this embodiment the aqueous suspension comprising nanoparticles or a suspension comprising nano-particles is first dried via a spray-drying process to obtain a nanoparticles containing powder and then melted via laser radiation in a powder bed fusion process, which is applied e.g. for 3D printing processes for producing drug containing solid dosage forms, to form an ASD.

In one embodiment of the method described herein the aqueous suspension comprises nano-particles having a solubility in the aqueous suspension of less than 10g/l, water and two polymers. In an alternative embodiment of the method described herein the aqueous suspension comprises nano-particles having a solubility in the aqueous suspension of less than 10g/l, water and three polymers.

In one embodiment of the method as described herein the nano-particles are active ingredient nano - particles.

As used herein the term "active ingredient" is used synonymous with the terms "active pharmaceutical ingredient", "active pharmaceutical agent" or "active drug substance" as used herein refers to an agent, active ingredient, compound, or substance, compositions, or mixtures thereof, that provide a pharmacological, often beneficial, effect.

In one embodiment of the method as described herein the aqueous suspension only consists of nanoparticles having a solubility of less than 10g/l, water and at least one polymer.

In an alternative embodiment of the method described herein the aqueous suspension comprising nanoparticles further comprises one or more surfactants.

Even in this embodiment the aqueous suspension comprising nano-particles does not comprise organic solvents but only nano-particles, e.g. nano-grinded active ingredient nano-particles, having a solubility of less than 10g/l, water, at least one matrix forming polymer and at least one surfactant as well as optionally one or more further components selected from the group consisting of an inert carrier material, formulation excipients, e.g. lactose, sugar types, cellulose types.

Suitable Surfactants can be selected from the group consisting of sodium dodecyl sulfate, dioctyl sulfosuccinate sodium (docusate sodium), disodium mono(2-ethylhexyl) sulfosuccinate, sodium desoxycholate, polysorbates.

In one embodiment of method described herein the aqueous suspension comprises nano-particles having a solubility in the aqueous suspension of less than 10g/l, water and two polymers and one or more surfactants. In an alternative embodiment of method described herein the aqueous suspension comprises nano-particles having a solubility in the aqueous suspension of less than 10g/l, water and three polymers and one or more surfactants.

In an alternative embodiment of the method described herein the suspension comprising nanoparticles further comprises one or more organic solvents. Examples of suitable organic solvents are ethanol, aceton, isopropanol, butanone and other solvent with ICH-3 classification and additionally a boiling point below the process temperature as defined above in the spray drying process description. If the suspension comprising nanoparticles further comprises one or more organic solvents it is not referred to as aqueous suspension comprising nanoparticles, but as suspension comprising nanoparticles. For sake of clarity the embodiments wherein the suspension comprising nanoparticles further comprises one or more organic solvents are explicitly mentioned herein, since it is clear that the method and especially the nano-dry melting process disclosed herein is also applicable for these cases i.e. it would be a method for the preparation of an amorphous solid dispersion comprising the steps of
- providing a suspension comprising nano-particles having a solubility in aqueous suspension of less than 10g/l, water, at least one organic solvent and at least one polymer,
- nano-dry-melting of the suspension comprising nano-particles for a time span between 0.1 seconds and 300 seconds, at a temperature between
   a) 20K below the glass transition temperature as determined with DSC according to DIN EN ISO 11357-2 at a heating rate of 10 K/min of the solid components of the suspension comprising nano-particles or 20K below the glass transition temperature of the at least one polymer, depending on what is higher,
   b) the decomposition temperature of the at least one polymer or the decomposition temperature of the nano-particles, depending which is lower
   to form the amorphous solid dispersion.

In a preferred embodiment of using one or more organic solvents the temperature is between
a) the glass transition temperature as determined with DSC according to DIN EN ISO 11357-2 at a heating rate of 10 K/min of the solid components of the suspension comprising nano-particles or the glass transition temperature of the at least one polymer, depending on what is higher,
b) the decomposition temperature of the at least one polymer or the decomposition temperature of the nano-particles, depending which is lower.

In an even more preferred embodiment of using one or more organic solvents the temperature is between
a) 20K above the glass transition temperature as determined with DSC according to DIN EN ISO 11357-2 at a heating rate of 10 K/min of the solid components of the suspension comprising nanoparticles or 20K above the glass transition temperature of the at least one polymer, depending on what is higher,
b) the decomposition temperature of the at least one polymer or the decomposition temperature of the nano-particles, depending which is lower.

In one embodiment of the method described herein the nano-dry-melting of the aqueous suspension comprising nano-particles occurs for a time span between 0.1 seconds and 180 seconds. In a preferred embodiment of the method described herein the nano-dry-melting of the aqueous suspension comprising nano-particles occurs for a time span between 0.1 seconds and 120 seconds and in an even more preferred embodiment of the method described herein the nano-dry-melting of the aqueous suspension comprising nano-particles occurs for a time span between 0.1 seconds and 60 seconds.

Likewise in those cases where a suspension comprising nanoparticles is processed the nano-dry-melting of the suspension comprising nano-particles occurs for a time span between 0.1 seconds and 300 seconds, preferably for a time span between 0.1 seconds and 180 seconds, more preferably for a time span between 0.1 seconds and 120 seconds and most preferred for a time span between 0.1 seconds and 60 seconds.

In one embodiment of the method described herein the process starts with the step of nanogrinding the particles to form nano-particles.

To a skilled person it is clear that if nano grinding is used in the method described herein said nano grinding can only be performed if the nano-particles are not completely dissolved. Hence the skilled person would choose a suitable temperature within the temperature range of between 253 K and 323 K, preferably between 273 K and 303 K for nano-grinding the nano-particles in the aqueous suspension.

Suitable methods of nano-grinding can be selected from the group consisting of wet bead milling in stirred media mills, wet bead milling in planetary mills - especially suitable for small amounts- and high pressure homogenization. Alternatively, the application of high shear forces in aqueous suspensions like in a high pressure homogenization process or the application of high impact forces between the particles like in a microfluidizer can be used to produce nano-particles.

Preferably the nanogrinding conditions are chosen in such a way that the ratio of active particles to (all) matrix forming polymers is ideally the ratio needed in the final ASD. Alternatively, for example in scenarios where this ratio would result in a suspension with undesirable characteristics in terms of viscosity etc.. However, polymer can be added following nanogrinding in order to reach ideal conditions e.g. with respect to viscosity etc..

In one example of the active ingredient is micronized by dry grinding, e.g. by means of an air jet mill (particle size: d90 < 100 µm). This process step is well established in the pharmaceutical industry and is well known to experts. This results in a standardized micronized starting material. The micronized active ingredient powder is then subjected to nano-grinding also termed wet grinding until the particle size distribution corresponds to the above values of d90 and d50. A stirred ball mill, a planetary mill or other mill suitable for the purpose of "nano-grinding" can be used as a mill as well as high pressure homogenization or microfluidizing processes as described above.

As described above in one embodiment for nano-grinding the active ingredient, water and at least one matrix-forming polymer are provided. Optionally, one or more surfactants can be added e.g. to facilitate production of a stable aqueous suspension comprising nano-particles or a suspension comprising nanoparticles. The preferred concentration of the ionic surfactant is below the respective critical micelle formation concentration. These components are dissolved before grinding in the amount of water used for grinding. The concentrations of the materials used for the grinding as well as the ratio of active ingredient and additional components are adjusted to optimize the grinding result, i.e. the particle size distribution should be as narrow as possible and the concentration of the materials during the grinding process should be as high as possible. The particle size distribution of the suspension can be measured by laser diffraction or dynamic light scattering methods. This ratio must be greater than or equal to the ratio in the later ASD, but in practice this is usually not a limitation. If the ratio of active ingredient and additional components is greater than desired in the later ASD, further additional component(s) is dissolved into the aqueous suspension comprising nano-particles or a suspension comprising nano-particles after grinding until the desired ratio is achieved. Grinding with a smaller quantity of auxiliary material is preferred, if the viscosity of the suspension would otherwise be too high for a good grinding result and the total concentration of the solids introduced would therefore have to be lowered. By reducing the quantity of additional components to a limit that does not negatively affect the grinding result, and adding the additional components later, the efficiency and economy of the process can be increased. At the end of this process an aqueous suspension comprising nano-particles or a suspension comprising nano-particles is obtained, which is then subjected to the next process step, nano-melt-drying.

High-pressure homogenization or even microfluidization can be used as an alternative to nano-grinding: First, the active ingredient is micronized by dry grinding in the same way (particle size: d90 < 100 µm) as in the established processes and thus the starting material is the same. The micronized active ingredient powder is then subjected to so many high-pressure homogenization cycles until the particle size distribution corresponds to the above-mentioned values of d90 and d50. There are also established process regulations for this, which are described in detail in the literature (DE4440337A1, WO9965469A2). With regard to the composition of the starting suspension with regard to active ingredient(s) and additional components and with regard to a possible addition of additional components after high-pressure homogenization, the same principles apply as described above for nano-grinding. At the end of this process, an aqueous suspension comprising nano-particles or a suspension comprising nano-particles is also obtained, which is then subjected to the next process step, nano-melt-drying.

Moreover a person skilled in the art is aware of the fact that further methods to manufacture nano-particles can be used as an alternative to nano-grinding: e.g. by precipitation of active ingredients which are dissolved in one or in mixtures of more than one organic solvents with an antisolvent, e.g. water (as described in WO2015/179334 or via using supercritical fluid technologies (M.J. Meziani et al., Supercritical Fluid Technology for Nanotechnology in Drug Delivery, M.M. de Villiers et al. (eds.), Nanotechnology in Drug Delivery, DOI: 10.1007/978-0-387-77667-5_3, pp. 69-104; WO2016/055696A1).

In one embodiment of the method described herein the generated ASD is further processed via tempering in order to facilitate homogenization of the solid components or relaxation of the at least one polymer.

Regardless of the device and method used for nano-melt-drying, the recovered ASD material can optionally be subjected to further processing steps. This can be for example a tempering step at elevated temperature, preferably in an inert gas atmosphere, to remove residual moisture and to achieve a more even distribution of the amorphous active ingredient in the polymer. However, post-treatment could also be a "fast" extrusion, which differs from the extrusion described above in that it can be carried out much faster (factor 3 or more) and thus with less temperature stress, since a dissolution process of the active ingredient into the polymer is no longer necessary but only homogeneously mixing. Such a "fast" extrusion could be used to facilitate an even more homogenous distribution of the active ingredient in the ASD.

Moreover, the further processing can accelerate polymer relaxation (e.g. via reducing the free volume in the polymer or increasing the intermolecular interaction between active ingredient and auxiliary material) and thus improve the storage stability of the ASD due to a lower probability of a recrystallization process in the ASD.

In one embodiment of the method described herein method is carried out continuously.

As used herein the term "continuous" refers to a method for carrying out at least two method steps in series in which the second method step begins processing before the first method step is completed.

For example, continuous processing can be achieved via connecting the exit of a nanogrinding device with the inlet of a drying device, thus allowing for a continuous process. Typically, in such a scenario further unit operations/and or devices can be applied like mixing, adding of further additives, granulation units. Since unit operations may work in different processing speed a buffer station like a buffer vessel may be employed.

In another aspect what is described herein relates to the use of the method as described above to generate a pharmaceutical formulation comprising a particulate pharmaceutical active ingredient which is coated with an at least partially water soluble polymer characterized in that the particulate pharmaceutical active ingredient is present in form of particles with a particle size distribution of d90< 1µm and further characterized in that within the polymer the same pharmaceutical active ingredient is also present in amorphous dispersed form and in that the total amount of the pharmaceutical active ingredient is larger than the amount of the pharmaceutical active ingredient soluble in the polymer at 20°C. Thus, the resulting pharmaceutical formulation comprises the active ingredient in amorphous form and in form of nanoparticles as described in patent application number EP19202455.2.

### FIGURES

Figure 1: XRPD-measurements of different Indomethacine-PVPK12 powders, produced with different drying temperatures.
Figure 2: XRPD-measurements of samples resulting from and suspensions comprising micro- (two upper curves) and nanoparticles (two lower curves processed at different temperature zones on Kofler bench.

### EXAMPLES

### Example 1: preparation of an aqueous suspension comprising nano-particles

The aqueous suspension comprising nano-particles was produced by means of a planetary ball mill (Fritsch Pulverisette 5). For this purpose, ~7 wt% indomethacin was stabilized with ~13 wt% PVP K12 and 0.2 wt% SDS. The polymer surfactant solution was prepared and dissolved separately in distilled water. Indomethacin powder was then added to the solution and the resulting suspension was homogenized on a stirring plate. The grinding chambers were filled to 60% (volume) with 0.4-0.6 mm (SiLibeads, zirconium oxide, yttrium stabilized) grinding media and the remaining volume with suspension, free of air bubbles. After 1 h 30 min grinding time at 400 rpm, a suspension comprising nanoparticles with particles d90 < 500 nm (Malvern, Mastersizer 2000) was available for production of ASD (drug load ~35 wt%) via nano melt drying.

### Example 2: Nano melt drying via spray drying

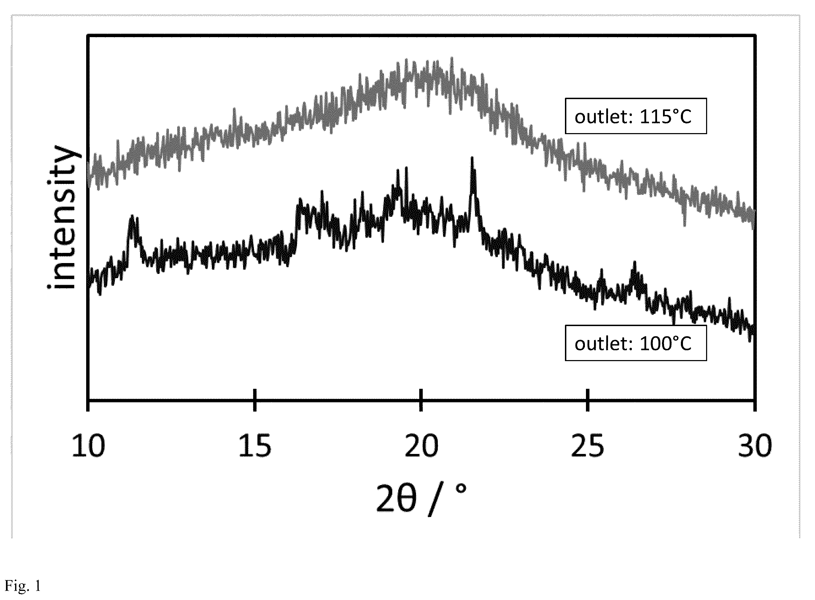

The aqueous suspension comprising nano-particles generated in example 1 was spray dried at two different temperatures to produce ASDs. For this process a Büchi B-290 labspray dryer was used. Two different inlet temperatures were chosen which were 200°C and 220°C. The resulting outlet temperatures during the process were 100°C and 115°C. Relative aspirator power and pump speed were fixed at 1 and 0.20. In Figure 1 the XRPD-graphs of both resulting powders is visible. By processing the suspension with an outlet temperature of 115°C it was possible to produce a completely X-ray amorphous ASD. For processing with 100°C outlet temperature the residence time was not sufficient to produce a completely X-ray amorphous ASD.

### Example 3: Comparison of suspensions comprising micro- and nanoparticles on Kofler-bench

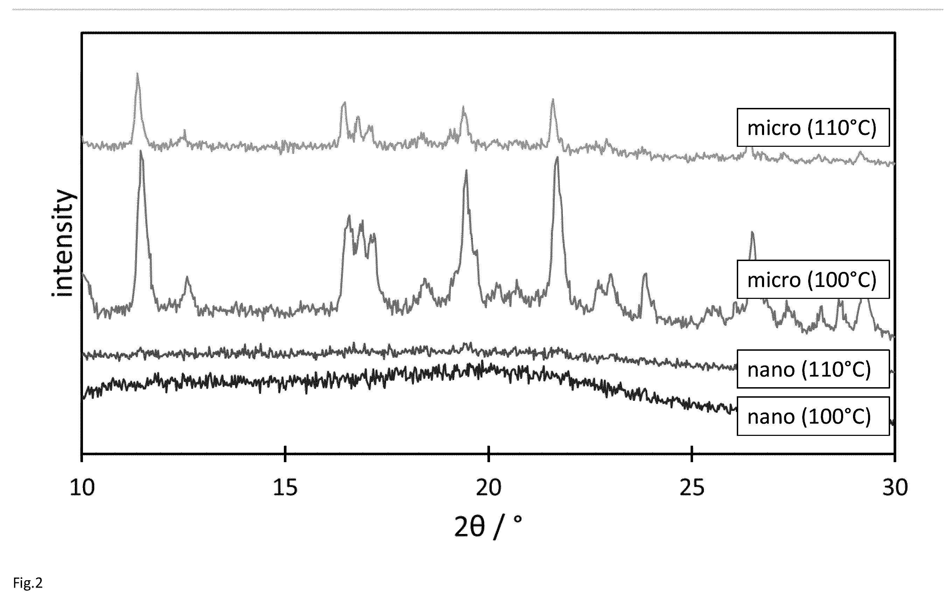

To compare the suspensions comprising microparticles used to produce the aqueous suspension comprising nano-particles with the resulting aqueous suspension comprising nano-particles a Kofler bench was used. There a film was extracted from the respective suspension and treated with different temperatures. The films were dried on Kofler bench for several minutes. Then samples of different temperature areas were taken and measured in XRPD-device. Figure 2 shows the different XPRD-graphs. It can be seen that in case of suspensions comprising nanoparticles fully amorphous ASD-films were produced. Films resulting from suspensions comprising microparticles are still crystalline. In addition, the films resulting from suspensions comprising nanoparticles were visually much more homogeneous than those from suspensions comprising microparticles.

## Claims

1. Method for the preparation of an amorphous solid dispersion (ASD) comprising the steps of
• providing an aqueous suspension at a temperature between 253 K und 323 K comprising nanoparticles having a solubility in the aqueous suspension of less than 10g/l, water and at least one polymer,
• nano-dry-melting of the aqueous suspension comprising nano-particles for a time span between 0.1 seconds and 300 seconds , at a temperature between
a) 20K below the glass transition temperature as determine with DSC according to DIN EN ISO 11357-2 at a heating rate of 10 K/min of the solid components of the aqueous suspension comprising nano-particles or 20K below the glass transition temperature of the at least one polymer, depending on what is higher,
b) the decomposition temperature of the at least one polymer or the decomposition temperature of the nano-particles, depending which is lower
to form the amorphous solid dispersion.

2. Method according to claim 1 wherein the nano-particles are active ingredient nano -particles.

3. Method according to claims 1-2, wherein the aqueous suspension only consists of nano-particles having a solubility of less than 10g/l, water and at least one polymer.

4. Method according to claims 1-2, wherein the aqueous suspension further comprises one or more surfactants.

5. Method according to anyone of the preceding claims wherein the nano-dry-melting temperature lies between
a) the glass transition temperature as determined with DSC according to DIN EN ISO 11357-2 at a heating rate of 10 K/min of the solid components of the aqueous suspension comprising nano-particles or the glass transition temperature of the at least one polymer, depending on what is higher,
b) the decomposition temperature of the at least one polymer or the decomposition temperature of the nano-particles, depending which is lower.

6. Method according to anyone of the preceding claims wherein the nano-dry-melting temperature lies between
a) 20 K above the glass transition temperature as determined with DSC according to DIN EN ISO 11357-2 at a heating rate of 10 K/min of the solid components of the aqueous suspension comprising nano-particles or 20 K above the glass transition temperature of the at least one polymer, depending on what is higher,
b) the decomposition temperature of the at least one polymer or the decomposition temperature of the nano-particles, depending which is lower.

7. Method according to anyone of the preceding claims wherein the nano-dry-melting of the aqueous suspension comprising nano-particles occurs for a time span between 0.1 seconds and 180 seconds.

8. Method according to claim 1 wherein the process starts with the step of nanogrinding the particles to form nano-particles.

9. Method according to claim 8 wherein the nano-grinding is carried out at a temperature between 273 K and 303 K.

10. Method according to anyone of the preceding claims wherein the nano-melt-drying is carried out via a process selected from the group consisting of spray drying, spray granulation, fluidized bed drying systems, contact drying, extrusion processes and drying by electromagnetic radiation.

11. Method according to anyone of the preceding claims wherein the generated ASD is further processed via tempering.

12. Method according to claim 8 wherein the method is carried out continuously.

## Patentansprüche

1. Verfahren zur Herstellung einer amorphen Feststoffdispersion (ASD), umfassend die Schritte
• Bereitstellen einer wässrigen Suspension bei einer Temperatur zwischen 253 K und 323 K, umfassend Nanopartikel mit einer Löslichkeit in der wässrigen Suspension von weniger als 10 g/l, Wasser und mindestens ein Polymer,
• Nano-Trockenschmelzen der wässrigen Suspension, umfassend Nanopartikel für eine Zeitspanne zwischen 0,1 Sekunden und 300 Sekunden, bei einer Temperatur zwischen
a) 20 K unterhalb der Glasübergangstemperatur, bestimmt mit DSC nach DIN EN ISO 11357-2 bei einer Heizrate von 10 K/min der festen Bestandteile der wässrigen Suspension, die Nanopartikel umfasst, oder 20 K unterhalb der Glasübergangstemperatur des mindestens einen Polymers, je nachdem, was höher ist,
b) die Zersetzungstemperatur des mindestens einen Polymers oder die Zersetzungstemperatur der Nanopartikel, je nachdem, was niedriger ist,
um die amorphe feste Dispersion zu bilden.

2. Verfahren nach Anspruch 1, wobei es sich bei den Nanopartikeln um Wirkstoff-Nanopartikel handelt.

3. Verfahren nach Anspruch 1-2, wobei die wässrige Suspension nur aus Nanopartikeln mit einer Löslichkeit von weniger als 10 g/l, Wasser und mindestens einem Polymer besteht.

4. Verfahren nach Anspruch 1-2, wobei die wässrige Suspension weiterhin ein oder mehrere Tenside umfasst.

5. Verfahren nach einem der vorangehenden Ansprüche, wobei die Nano-Trockenschmelztemperatur zwischen
a) der Glasübergangstemperatur, bestimmt mit DSC nach DIN EN ISO 11357-2 bei einer Heizrate von 10 K/min der festen Bestandteile der wässrigen Suspension, die Nanopartikel umfasst, oder der Glasübergangstemperatur des mindestens einen Polymers, je nachdem, was höher ist
b) der Zersetzungstemperatur des mindestens einen Polymers oder der Zersetzungstemperatur der Nanopartikel, je nachdem, welche niedriger ist liegt.

6. Verfahren nach einem der vorhergehenden Ansprüche, wobei die Nano-Trockenschmelztemperatur zwischen
a) 20 K über der mit DSC nach DIN EN ISO 11357-2 bei einer Heizrate von 10 K/min bestimmten Glasübergangstemperatur der festen Bestandteile der Nanopartikel umfassenden wässrigen Suspension oder 20 K über der Glasübergangstemperatur des mindestens einen Polymers, je nachdem, welcher Wert höher ist,
b) die Zersetzungstemperatur des mindestens einen Polymers oder die Zersetzungstemperatur der Nanopartikel, je nachdem, welcher Wert niedriger ist liegt

7. Verfahren nach einem der vorhergehenden Ansprüche, wobei das Nano-Trockenschmelzen der wässrigen Suspension, die Nanopartikel umfasst, über eine Zeitspanne zwischen 0,1 Sekunden und 180 Sekunden erfolgt.

8. Verfahren nach Anspruch 1, wobei das Verfahren mit dem Schritt des Nanomahlens der Partikel zur Bildung von Nanopartikeln beginnt.

9. Verfahren nach Anspruch 8, wobei die Nanomahlung bei einer Temperatur zwischen 273 K und 303 K durchgeführt wird.

10. Verfahren nach einem der vorhergehenden Ansprüche, wobei die Nano-Schmelztrocknung mittels eines Verfahrens durchgeführt wird, das aus der Gruppe bestehend aus Sprühtrocknung, Sprühgranulation, Wirbelschichttrocknungssystemen, Kontakttrocknung, Extrusionsverfahren und Trocknung durch elektromagnetische Strahlung ausgewählt ist.

11. Verfahren nach einem der vorhergehenden Ansprüche, wobei das erzeugte ASD durch Tempern weiterbehandelt wird.

12. Verfahren nach Anspruch 8, wobei das Verfahren kontinuierlich durchgeführt wird.

## Revendications

1. Méthode de préparation d'une dispersion solide amorphe (DSA) comprenant les étapes suivantes :
∘ Fournir une suspension aqueuse à une température comprise entre 253 K et 323 K comprenant des nanoparticules ayant une solubilité dans la suspension aqueuse inférieure à 10g/l, de l'eau et au moins un polymère.
∘ Nano-séchage-fusion de la suspension aqueuse comprenant des nanoparticules pendant une durée comprise entre 0,1 seconde et 300 secondes, à une température comprise entre:
▪ 20K en dessous de la température de transition vitreuse déterminée par DSC selon DIN EN ISO 11357-2 à un taux de chauffage de 10 K/min des composants solides de la suspension aqueuse comprenant des nanoparticules ou 20K en dessous de la température de transition vitreuse du ou des polymères, selon ce qui est le plus élevé.
▪ La température de décomposition du ou des polymères ou la température de décomposition des nanoparticules, selon ce qui est le plus bas.
pour former la dispersion solide amorphe.

2. Méthode selon la revendication 1, où les nanoparticules sont des nanoparticules d'ingrédient actif.

3. Méthode selon les revendications 1-2, où la suspension aqueuse ne consiste qu'en des nanoparticules ayant une solubilité inférieure à 10g/l, de l'eau et au moins un polymère.

4. Méthode selon les revendications 1-2, où la suspension aqueuse comprend en outre un ou plusieurs surfactants.

5. Méthode selon l'une quelconque des revendications précédentes, où la température de nano-séchage-fusion se situe entre:
∘ a) La température de transition vitreuse déterminée par DSC selon DIN EN ISO 11357-2 à un taux de chauffage de 10 K/min des composants solides de la suspension aqueuse comprenant des nanoparticules ou la température de transition vitreuse du ou des polymères, selon ce qui est le plus élevé,
∘ b) La température de décomposition du ou des polymères ou la température de décomposition des nanoparticules, selon ce qui est le plus bas.

6. Méthode selon l'une quelconque des revendications précédentes, où la température de nano-séchage-fusion se situe entre:
∘ a) 20 K au-dessus de la température de transition vitreuse déterminée par DSC selon DIN EN ISO 11357-2 à un taux de chauffage de 10 K/min des composants solides de la suspension aqueuse comprenant des nanoparticules ou 20 K au-dessus de la température de transition vitreuse du ou des polymères, selon ce qui est le plus élevé.
∘ b) La température de décomposition du ou des polymères ou la température de décomposition des nanoparticules, selon ce qui est le plus bas.

7. Méthode selon l'une quelconque des revendications précédentes, où le nano-séchage-fusion de la suspension aqueuse comprenant des nanoparticules se produit pendant une durée comprise entre 0,1 seconde et 180 secondes.

8. Méthode selon la revendication 1, où le processus commence par l'étape de nano-broyage des particules pour former des nanoparticules.

9. Méthode selon la revendication 8, où le nano-broyage est effectué à une température comprise entre 273 K et 303 K.

10. Méthode selon l'une quelconque des revendications précédentes, où le nano-séchage-fusion est effectué via un processus sélectionné parmi le groupe comprenant le séchage par pulvérisation, la granulation par pulvérisation, les systèmes de séchage en lit fluidisé, le séchage par contact, les processus d'extrusion et le séchage par rayonnement électromagnétique.

11. Méthode selon l'une quelconque des revendications précédentes, où la DSA générée est en outre traitée par trempe.

12. Méthode selon la revendication 8, où la méthode est réalisée en continu.
